# EUROPÄISCHE PATENTSCHRIFT

(11) **EP 1 466 563 B1**
(45) Veröffentlichungstag und Bekanntmachung des Hinweises auf die Patenterteilung: **16.11.2005**
(21) Anmeldenummer: 03007084.1
(22) Anmeldetag: 28.03.2003
(51) Int. Cl.: A61B 17/34

(54) **Dilatationssystem**
Dilatation system
Système de dilatation

(43) Veröffentlichungstag der Anmeldung: 13.10.2004
(73) Patentinhaber: Richard Wolf GmbH, 75438 Knittlingen (DE)
(72) Erfinder: Wagner, Carl-Sebastian, Dipl.-Ing., 75015 Bretten (DE); Weigel, Manfred, 75177 Pforzheim (DE)
(74) Vertreter: Hemmer, Arnd

(56) Entgegenhaltungen:
- US-A- 4 687 469
- US-A- 5 431 676
- US-A- 5 827 319
- US-A1- 2002 091 405

## Beschreibung

Die Erfindung betrifft ein Dilatationssystem.

Eine Dilatation einer Öffnung zu einer Körperhöhle ist erforderlich, wenn ein bestehender bzw. zuerst erzeugter Zugang mit kleinerem Querschnitt durch einen Zugang größeren Querschnitts ersetzt werden soll. Beispielsweise soll zunächst eine Veresskanüle oder Trokarhülse eingeführt und später unter Erweiterung des bereits geschaffenen Gewebedurchgangs eine größere Trokar- oder Instrumentenhülse eingeführt werden, um endoskopische Eingriffe vornehmen zu können. Dabei soll eine Erweiterung des bereits geschaffenen Gewebedurchganges mittels Dilatation oder Vergrößerung der Schnittincision erfolgen, ohne dass der bereits geschaffene Gewebedurchgang verloren geht. Wenn jedoch das zuerst eingeführte Instrument, beispielsweise eine Trokarhülse mit geringem Durchmesser, entnommen wird, um anschließend beispielsweise einen Dilatationsdorn zum Aufweiten des Gewebedurchganges einzuführen, besteht die Gefahr, dass nach Entnahme des Instrumentes sich der Gewebedurchgang wieder schließt und nicht mehr auffindbar ist.

Beispielsweise aus US 5,431,676, US 6,080,174 und US 6,325,812 sind Trokarhülsen bekannt, deren Schaft aus einem im Durchmesser veränderbaren Geflecht besteht. Dieses Geflecht kann sich aufdehnen, um einen größeren, erweiterten Zugang zu schaffen. Nachteilig bei diesen Anordnungen ist, dass die Wandstärke einer solchen Trokarhülse beim Einbringen bspw. einer Veresskanüle den Gesamtdurchmesser deutlich vergrößert. Ferner ist eine solche Trokarhülse aus einem Geflecht teuer in der Herstellung, schlecht zu reinigen und erfodert beim Einbringen von bspw. einer Veresskanüle einen erhöhten Kraftaufwand.

Es ist daher Aufgabe der Erfindung, ein verbessertes Dilatationssystem zu schaffen, durch welches der Gesamtdurchmesser beim Einführen eines endoskopischen Instrumentes nur geringfügig vergrößert wird und welches kostengünstig herzustellen und leicht zu reinigen ist.

Diese Aufgabe wird durch ein Dilatationssystem mit den im Anspruch 1 angegebenen Merkmalen gelöst. Bevorzugte Ausführungsformen ergeben sich aus den Unteransprüchen.

Das erfindungsgemäße Dilatationssystem umfasst als wesentliche Elemente einen Dilatationsdorn sowie eine rohrförmige Führungshülse. Dabei weist der Dilatationsdorn einen Durchmesser auf, welcher vom distalen Ende her in proximaler Richtung zunimmt, so dass das distale Ende als relativ dünne Führungsnase ausgebildet ist. Der Durchmesser der Führungsnase des Dilatationsdornes entspricht im Wesentlichen dem Innendurchmesser der rohrförmigen Führungshülse, so dass der Dilatationsdorn mit seinem schmalen bzw. im Durchmesser reduzierten Ende leicht in die Führungshülse eingeführt werden kann. Zusätzlich kann der Dilatationsdorn an seinem distalen Ende abgerundet oder angefast ausgebildet sein, um ein leichteres Einführen in die Führungshülse zu ermöglichen. Die Führungshülse ist trotz relativ geringer Wandstärke derart eigenstabil ausgebildet, dass sie, wenn sie in eine Öffnung, beispielsweise eine Öffnung in der Bauchdecke, eingesetzt ist, diese Öffnung erhalten kann, ohne dass weitere Elemente im Inneren der Führungshülse angeordnet werden müssen. Ferner ist die Führungshülse so ausgebildet, dass sie über ihre gesamte Länge entlang zumindest einer Linie auftrennbar ist. Dies ermöglicht die Entnahme der Führungshülse, nachdem der Dilatationsdorn in die Körperöffnung eingeführt ist. Das erfindungsgemäße Dilatationssystem ermöglicht die Durchführung eines neuen Dilatationsverfahrens, bei welchem zunächst die Führungshülse gemeinsam mit einem endoskopischen Instrument wie bspw. einer Veresskanüle oder dgl. in das Gewebe eingeführt wird, um zunächst eine Öffnung bzw. einen Zugang mit kleinem Durchmesser zu schaffen. Nach der anschließenden Entnahme des endoskopischen Instrumentes hält die eingeführte Führungshülse die geschaffene Öffnung offen, so dass nun in die Führungshülse der Dilatationsdorn gemeinsam mit einer diesen umgebenden Trokarhülse oder dgl. eingeführt werden kann. Dabei weitet der Dilatationsdorn aufgrund seines in proximaler Richtung zunehmenden Durchmessers die Führungshülse und die geschaffene Öffnung auf. Gleichzeitig wird die Führungshülse entlang zumindest einer Linie über ihre gesamte Länge aufgetrennt. Ist der Zugang über seine gesamte Länge vollständig dilatiert und die Führungshülse über ihre gesamte Länge aufgetrennt ist, kann diese aus dem geschaffenen, erweiterten Zugang entnommen werden, wobei dieser dann durch den eingeführten Dilatationsdorn bzw. durch die diesen umgebende Trokarhülse offen gehalten wird. Die Führungshülse ist als Wegwerfteil ausgebildet, wodurch sowohl der Reinigungsaufwand als auch die Kosten deutlich reduziert werden.

Bevorzugt weist die Führungshülse zumindest eine sich in Längsrichtung der Führungshülse vorzugsweise über deren gesamte Länge erstreckende Sollbruchstelle auf. Eine solche Sollbruchstelle gewährleistet, dass die Führungshülse beim Aufweiten durch den Dilatationsdorn entlang einer vorgegebenen, definierten Linie aufreißt.

Ferner weist das Dilatationssystem eine Veresskanüle mit einem Außendurchmesser auf, der dem Innendurchmesser der Führungshülse entspricht. Dabei kann die Veresskanüle eine Trokarhülse aufweisen bzw. im Inneren einer Trokarhülse angeordnet sein, deren Außendurchmesser dem Innendurchmesser der Führungshülse entspricht. Mit einer solchen Veresskanüle kann der Zugang zur Körperhöhle vorzugsweise unter optischer Kontrolle geschaffen werden. Dabei ermöglicht die Abstimmung des Innendurchmessers der Führungshülse und des Außendurchmessers der Veresskanüle, dass diese gemeinsam in das Gewebe eingeführt werden können. Dazu wird die Führungshülse zuerst auf die Veresshülse aufgeschoben und dann gemeinsam mit dieser in das Gewebe eingestochen. Anschließend kann bzw. die Veresskanüle proximalwärts aus der Führungshülse herausgezogen werden, so dass allein die Führungshülse in dem geschaffenen Zugang bzw. der geschaffenen Öffnung verbleibt und diesen bis zur Einführung des Dilatationsdornes erhält. Anstelle einer Veresskanüle kann auch ein anderes entsprechend geeignetes Instrument zum Schaffen des Zugangs verwendet werden.

Weiter bevorzugt sind in der Führungshülse zwei vorzugsweise diametral entgegengesetzt angeordnete Sollbruchstellen ausgebildet, welche sich in Längsrichtung der Führungshülse erstrecken. Diese Ausgestaltung ermöglicht, dass die Führungshülse beim Aufweiten durch den Trokardorn in zwei Teile zerfällt und anschließend leichter aus dem geschaffenen Zugang entnommen werden kann. Es können auch mehr als zwei Sollbruchstellen über den Umfang verteilt vorgesehen werden, wobei die Führungshülse dann beim Aufweiten in drei oder mehr Teile zerfällt und entsprechend zwischen Trokarhülse und Gewebe herausgezogen werden kann.

Die Sollbruchstellen sind bevorzugt durch Perforation oder bereichsweise schwächeres Material der Führungshülse ausgebildet. Das Material der Führungshülse kann an vorgegebenen Stellen beispielsweise dadurch geschwächt sein, dass es dünner ausgebildet ist, d. h. die Führungshülse an den entsprechenden Stellen eine dünnere Wandstärke aufweist.

Gemäß einer speziellen Ausführungsform ist die Führungshülse aus zumindest zwei voneinander getrennten, ineinander angeordneten Hülsen ausgebildet, welche jeweils zumindest einen sich in Längsrichtung der Führungshülse vorzugsweise über deren gesamte Länge erstreckenden Schlitz oder eine sich entsprechend erstreckende Sollbruchstelle aufweisen, wobei die Schlitze oder Sollbruchstellen in den beiden Hülsen zueinander umfänglich versetzt angeordnet sind. Diese Anordnung bewirkt, dass beim Aufweiten durch den Dilatationsdorn beide Hülsen der Führungshülse entlang des Schlitzes bzw. der Sollbruchstelle aufreißen bzw. aufweiten, wobei sich der entstehende Schlitz verbreitert. Durch die versetzte Anordnung der Sollbruchstellen bzw. Schlitze über den Umfang der Führungshülse wird erreicht, dass der in der einen Hülse entstehende Schlitz jeweils von der Wandung der anderen Hülse überdeckt wird, so dass die Führungshülse auch beim Aufweiten stets umfänglich vollständig geschlossen ist. Dadurch ist über den gesamten Umfang des Dilatationsdornes stets eine Wandung der Führungshülse angeordnet, wodurch eine sichere Führung des Dilatationsdornes gewährleistet ist und die Führungshülse dennoch leicht aufreißt bzw. aufzuweiten ist, wenn der Dilatationsdorn in die Führungshülse eingeführt wird. Wenn die Führungshülse aus zwei ineinander angeordneten Hülsen besteht, sind die in den beiden Hülsen ausgebildeten Schlitze bzw. Sollbruchstellen vorzugsweise um 180° versetzt zueinander angeordnet. Anstelle zweier Hülsen können auch drei oder mehr Hülsen eingesetzt werden. Wenn die Führungshülse beispielsweise aus drei Hülsen aufgebaut ist, sind deren Schlitze vorzugsweise jeweils um 120° zueinander versetzt am Umfang der Führungshülse angeordnet, so dass jeder Schlitz von der Wandung einer anderen Hülse überdeckt wird. Die ineinander angeordneten Hülsen sind bevorzugt so ausgebildet, dass die jeweils innere Hülse mit ihrem Außenumfang jeweils am Innenumfang der nächsten äußeren Hülse anliegt, so dass zwischen den einzelnen Hülsen der Führungshülse im Wesentlichen kein Freiraum bzw. Spiel vorhanden ist.

Bevorzugt ist am proximalen Ende der Führungshülse zumindest ein Halteelement ausgebildet. Ein solches Halteelement, z. B. ein Haltering, erleichtert die Handhabung der Führungshülse. So kann die Führungshülse beim Einschieben des Dilatationsdorns an dem Haltering festgehalten werden, um zu verhindern, dass die Führungshülse von dem Dilatationsdorn in die Körperhöhle geschoben wird. Ferner kann die Führungshülse nach dem Aufweiten durch den Dilatationsdorn leicht an dem Haltering erfasst werden und aus dem geschaffenen Zugang herausgezogen werden. Anstelle eines Halteringes kann auch ein anderes geeignetes Griffelement an der Führungshülse vorgesehen sein.

Die Führungshülse ist zweckmäßigerweise aus einem vorzugsweise transparenten Kunststoff gefertigt. Aus Kunststoff kann die Führungshülse kostengünstig als Wegwerfteil gefertigt werden. Ferner lassen sich in Kunststoff leicht die geforderten Sollbruchstellen ausbilden. Transparenter Kunststoff ist besonders geeignet, um die visuelle Kontrolle beim Einführen der Führungshülse und der einzelnen Instrumente zu ermöglichen. Geeignete Kunststoffe sind Polymere wie z. B. PE, PTFE, PP, FEP etc.

Weiter bevorzugt weist der Dilatationsdorn zumindest eine Schneide zum Auftrennen der Führungshülse auf. Eine solche Schneide erstreckt sich zweckmäßigerweise in Längsrichtung des Dilatationsdornes und schneidet beim Einführen in die Führungshülse diese auf. Bevorzugt greift die Schneide dabei in die Sollbruchstelle der Führungshülse ein und trennt diese auf. Es sind jedoch auch Ausführungsformen möglich, bei denen die Führungshülse keine Sollbruchstelle aufweist und allein durch die an dem Dilatationsdorn vorgesehene Schneide in ihrer Längsrichtung aufgetrennt wird.

Ferner betrifft die Erfindung eine Führungshülse für ein Dilatationssystem, wie es vorangehend beschrieben worden ist. Wie beschrieben weist eine derartige Führungshülse erfindungsgemäß eine sich in Längsrichtung der Führungshülse vorzugsweise über deren gesamte Länge erstreckende Sollbruchstelle auf. Diese ermöglicht ein Aufreißen bzw. Aufweiten der Führungshülse, wenn ein Dilatationsdorn in die Führungshülse eingeführt wird. Die Ausgestaltung der Führungshülse entspricht dabei dem oben beschriebenen Aufbau und bevorzugten Ausführungsformen der Führungshülse.

Nachfolgend wird die Erfindung beispielhaft anhand der beigefügten Figuren beschrieben. In diesen zeigt:
- Fig. 1: eine Seitenansicht der Führungshülse mit eingesetzter Trokarhülse und Veresskanüle
- Fig. 2: eine vergrößerte Ansicht des distalen Endes der Führungshülse im eingestochenen Zustand,
- Fig. 3: schematisch das Einsetzen eines Dilatationsdornes in die Führungshülse,
- Fig. 4: den Dilatationsdorn beim Einschieben in die Führungshülse,
- Fig. 5: das Aufreißen der Führungshülse beim Einschieben des Dilatationsdornes,
- Fig. 6: die Führungshülse im aufgetrennten Zustand mit einer eingesetzten, größeren Trokarhülse,
- Fig. 7: das Entnehmen der aufgetrennten Führungshülse,
- Fig. 8a und Fig. 8b: eine erste Ausführungsform der Führungshülse in einer Schnitt- und Seitenansicht,
- Fig. 9a und Fig. 9b: eine zweite Ausführungsform der Führungshülse in einer Schnitt- und Seitenansicht,
- Fig. 10: eine Schnittansicht einer dritten Ausführungsform der Führungshülse,
- Fig. 11: eine Schnittansicht der Führungshülse gemäß Fig. 10 im aufgeweiteten Zustand und
- Fig. 12: eine Seitenansicht eines Dilatationsdornes mit Schneide

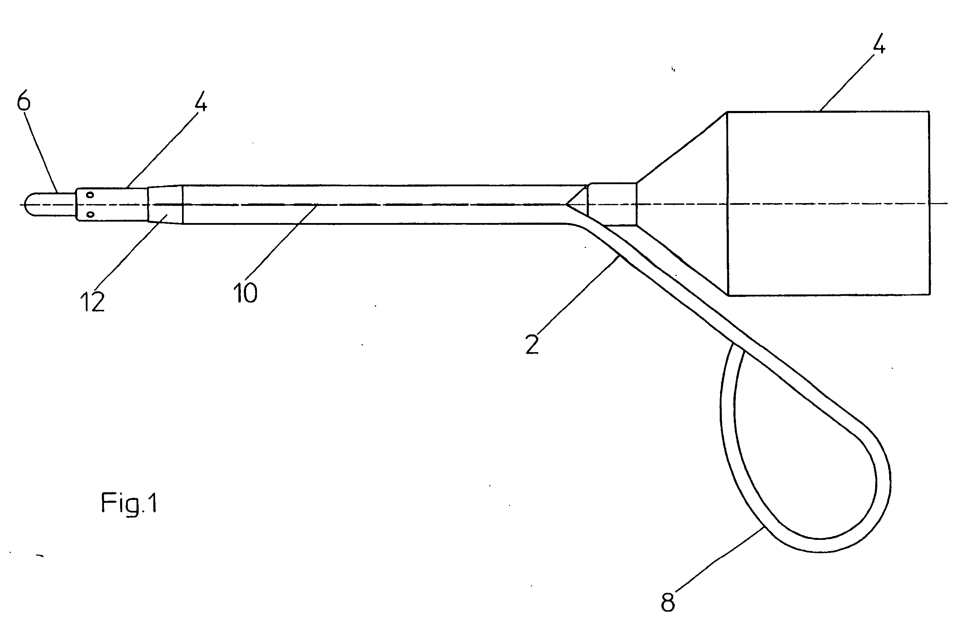

Fig. 1 zeigt eine Seitenansicht einer Führungshülse 2, in die eine Trokarhülse 4 eingesetzt ist. Die Trokarhülse 4 weist in ihrem Inneren eine am distalen Ende austretende optische Veresskanüle 6 auf. Die Führungshülse weist an ihrem proximalen Ende einen Haltering 8 auf, welcher zur besseren Handhabung der Führungshülse 2 dient. Ferner weist die Führungshülse 2 in ihrer Wandung eine linienförmige Sollbruchstelle 10 in Form einer Perforation auf, welche sich in Längsrichtung der Führungshülse erstreckt. Der Innendurchmesser der Führungshülse 2 entspricht im Wesentlichen dem Außendurchmesser der Trokarhülse 4, so dass die Führungshülse 10 zum Einführen in das Gewebe auf die Trokarhülse 4 aufgeschoben werden kann. An ihrem distalen Ende weist die Führungshülse 2 eine konische Anschrägung 12 auf, welche das Einführen in das Gewebe erleichtern soll.

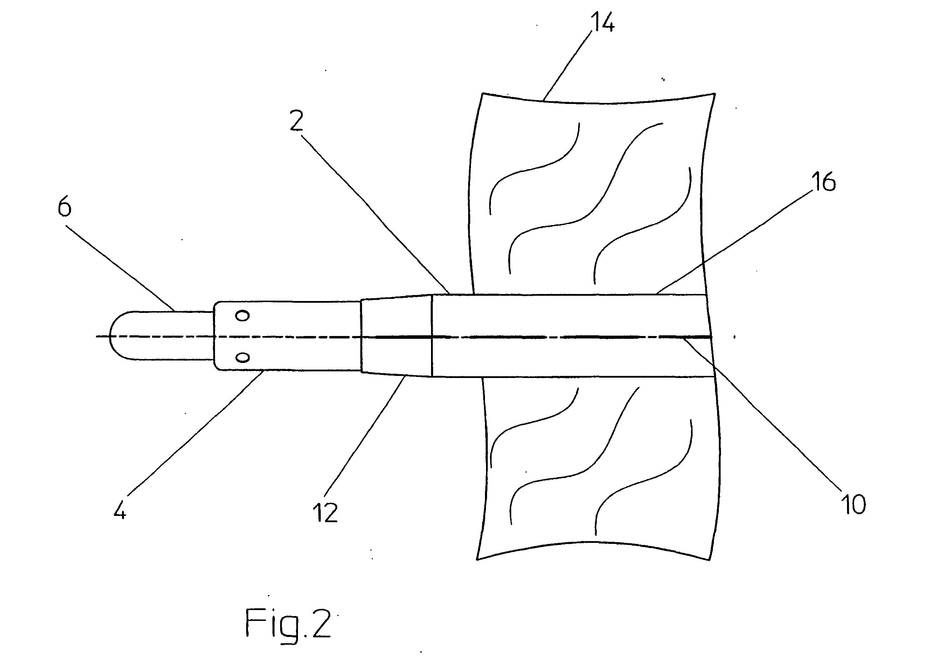

Das Einführen der Trokarhülse mit der aufgeschobenen Führungshülse 2 in das Gewebe wird anhand von Fig. 2 näher erläutert. Fig. 2 zeigt schematisch einen Schnitt durch die Bauchdecke 14, in welche die Trokarhülse 4 mit der aufgeschobenen Führungshülse 2 eingestochen ist. Beim Einstechen wird durch die optische Veresskanüle 6 eine optische Kontrolle ermöglicht. Beim Einstechen der Trokarhülse 4 in das Gewebe 14 wird die Führungshülse 2 gleichzeitig mit in das Gewebe 14 eingeführt, so dass die Führungshülse 2 in der geschaffenen Öffnung 16 im Gewebe zu liegen kommt.

Nach dem Einstechen und Einführen der Führungshülse 2 in das Gewebe 14 mit Hilfe der Trokarhülse 4 werden die Trokarhülse 4 und die Veresskanüle 6 gemeinsam aus der Führungshülse 2 proximalwärts herausgezogen, so dass nur die Führungshülse 2 in der Öffnung 16 in dem Gewebe 14 verbleibt und die Öffnung 16 erhält.

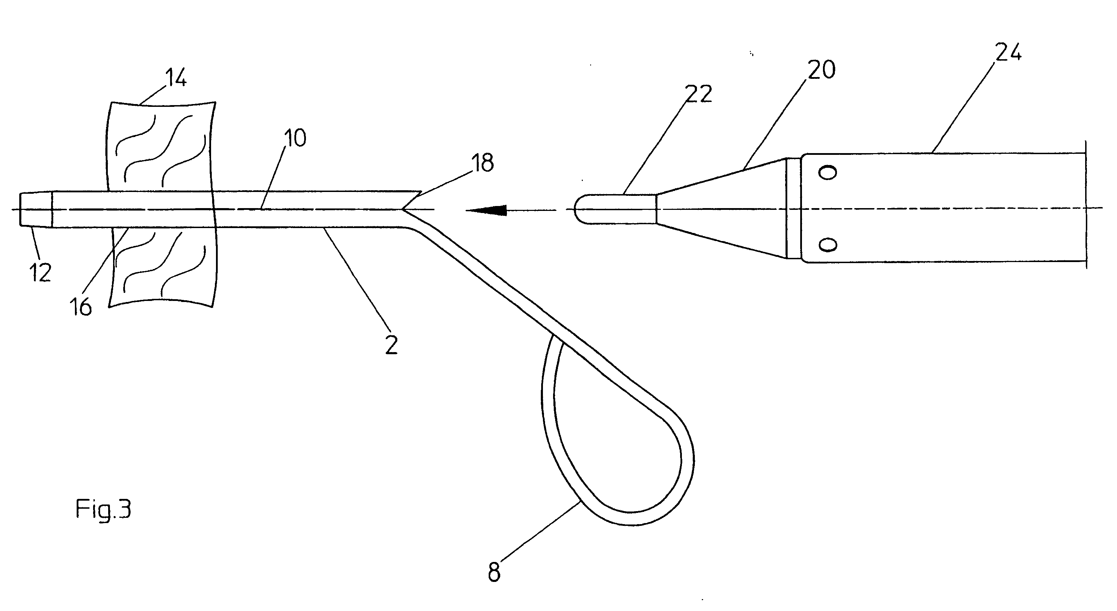

Fig. 3 zeigt den Zustand, in welchem Trokarhülse 4 und Veresskanüle 6 bereits in proximaler Richtung aus der Führungshülse 2 herausgezogen worden sind. Soll nun die geschaffene Öffnung 16 erweitert werden, um beispielsweise eine größere Trokarhülse für einen endoskopischen Eingriff einführen zu können, wird in die proximalseitige Öffnung 18 der Führungshülse 2 ein Dilatationsdorn 20 eingeführt. Der Dilatationsdorn 20 ist kegelförmig ausgebildet, so dass sich sein Durchmesser vom distalen Ende in proximaler Richtung erweitert. Am distalen Ende weist der Dilatationsdorn 20 eine Führungsnase 22 mit kleinem Durchmesser auf. Dabei ist der Durchmesser der Führungsnase 22 auf den Innendurchmesser der Führungshülse 2 abgestimmt, so dass die Führungsnase 22 leicht in die Führungshülse 2 eingeführt und in dieser geführt werden kann. Zusätzlich ist die Führungsnase 22 an ihrem distalen Ende atraumatisch ausgebildet, um dadurch dem Verlauf der Führungshülse ungehindert folgen zu können. Der Dilatationsdorn 20 ist im Inneren einer Trokarhülse 24 mit größerem Durchmesser angeordnet. Die Trokarhülse 24 weist einen größeren Durchmesser als die Trokarhülse 4 auf, was ein Aufweiten des Gewebes 14 erforderlich macht, um die Trokarhülse 24 in eine Körperhöhle einführen zu können. Wie in Fig. 3 durch den Pfeil angedeutet, wird der Dilatationsdorn 20 mit der aufgesetzten Trokarhülse 24 in distaler Richtung in die Führungshülse 2 von deren proximalen Ende her eingeführt.

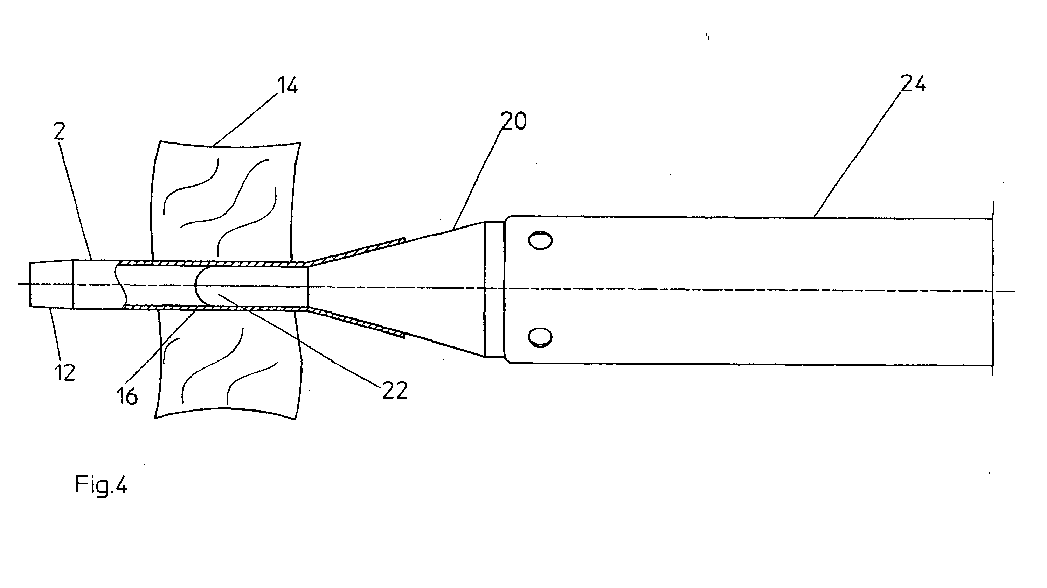

Fig. 4 zeigt den Zustand, in welchem die Führungsnase 22 des Dilatationsdornes 20 in die Führungshülse 2 eintritt. Die Führungsnase 22 dient zum Einführen des Dilatationsdornes 20 in die Führungshülse 2 und der sicheren Führung durch das Gewebe. Der kegelige, sich proximalwärts erweiternde Abschnitt des Dilatationsdornes 20 tritt im Anschluss an die Führungsnase 22 in die Führungshülse 2 ein und weitet diese auf.

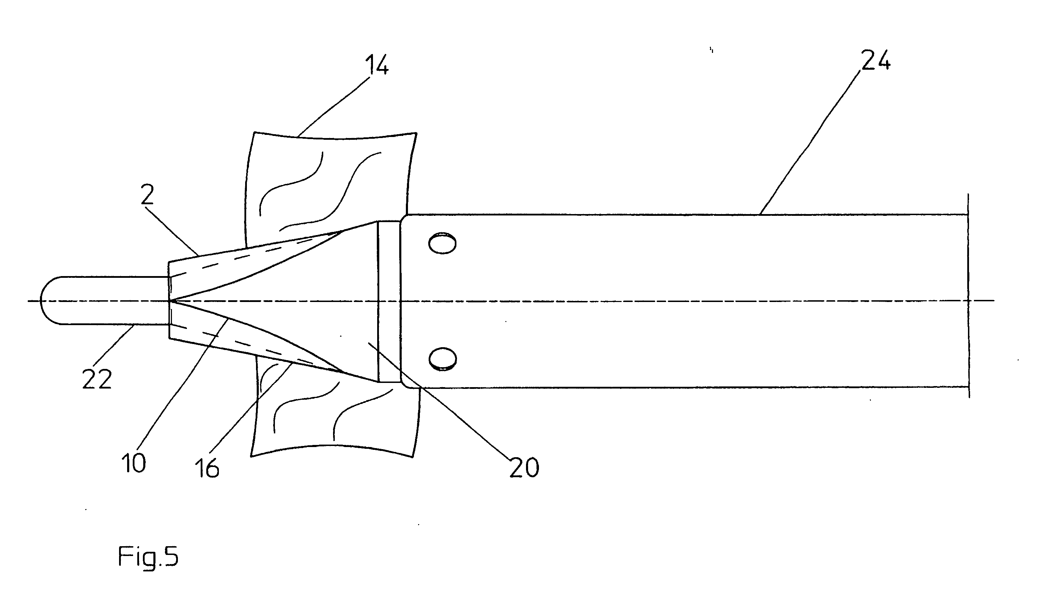

Beim weiteren Einschieben des Dilatationsdornes 20 in distaler Richtung wird, wie in Fig. 5 dargestellt, die Öffnung 16 in dem Gewebe 14 ebenfalls aufgeweitet. Dabei reißt die Führungshülse 2, wie in Fig. 5 gezeigt, entlang ihrer Sollbruchstelle bzw. Sollbruchstellen 10 linienförmig über die gesamte Länge auf. Dies ermöglicht, dass sich die Führungshülse 2 entsprechend erweitert bzw. aufklappt, so dass ein Eintritt des Teils des Dilatationsdornes 20 mit größerem Durchmesser und anschließend der Trokarhülse 24 in die Öffnung 16 ermöglicht wird. Die Führungshülse 2 wird beim Einschieben des Dilatationsdorns 20 an dem Haltering 8 festgehalten, um ein Verschieben in die Körperhöhle durch den Dilatationsdorn 20 zu verhindern.

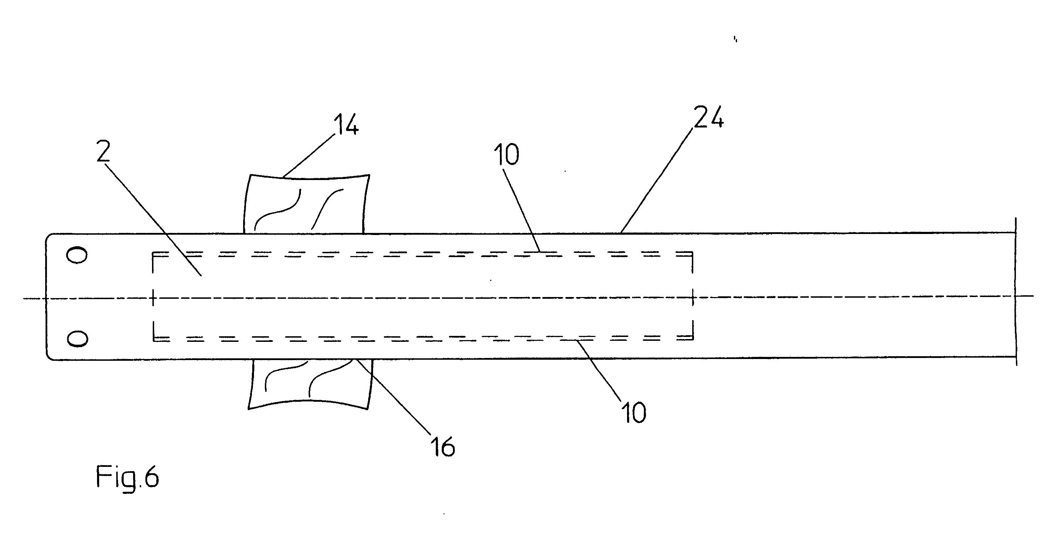

Fig. 6 zeigt den Zustand, in dem die Trokarhülse 24 vollständig durch das Gewebe 14 hindurchgeführt ist und der Dilatationsdorn 20 aus der Trokarhülse 24 entnommen ist. Der Dilatationsdorn 20 wird aus der Trokarhülse 24 in proximaler Richtung herausgezogen, so dass die Trokarhülse 24 dabei in der geschaffenen Öffnung 16 in dem Gewebe 14 verbleibt. Durch die Trokarhülse 24 können dann weitere Instrumente zur Durchführung eines endoskopischen Eingriffs hindurch in die Körperhöhle eingeführt werden. In dem in Fig. 6 gezeigten Zustand ist die Führungshülse 2 über ihre gesamte Länge aufgerissen und liegt nun an einem Umfangsabschnitt der Trokarhülse 24 zwischen Trokarhülse 24 und Gewebe 14.

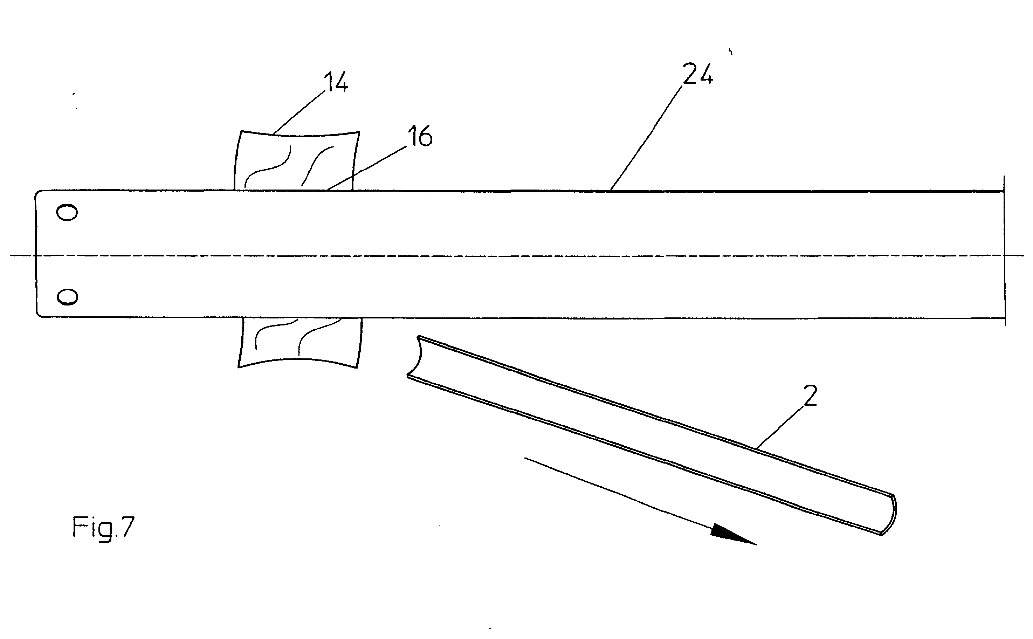

Im nächsten Schritt, welcher in Fig. 7 gezeigt ist, wird die aufgerissene Führungshülse 2 proximalwärts zwischen Trokarhülse 24 und Gewebe 14 herausgezogen, wobei sie am in Fig. 7 nicht gezeigten Haltering 8 ergriffen werden kann. Die Trokarhülse 24 verbleibt dabei in der geschaffenen Öffnung 16 in dem Gewebe 14.

Das zuvor beschriebene Verfahren, welches durch das erfindungsgemäße Dilatationssystem insbesondere die Führungshülse 2 ermöglicht wird, gewährleistet, dass die im Gewebe geschaffene Öffnung 16 erhalten bleibt, um nach erfolgten Entnahme der Veresskanüle 6 und der Trokarhülse 4 mit geringerem Durchmesser aus dem Gewebe die Dilatation zum Einführen der größeren Trokarhülse 24 ausführen zu können.

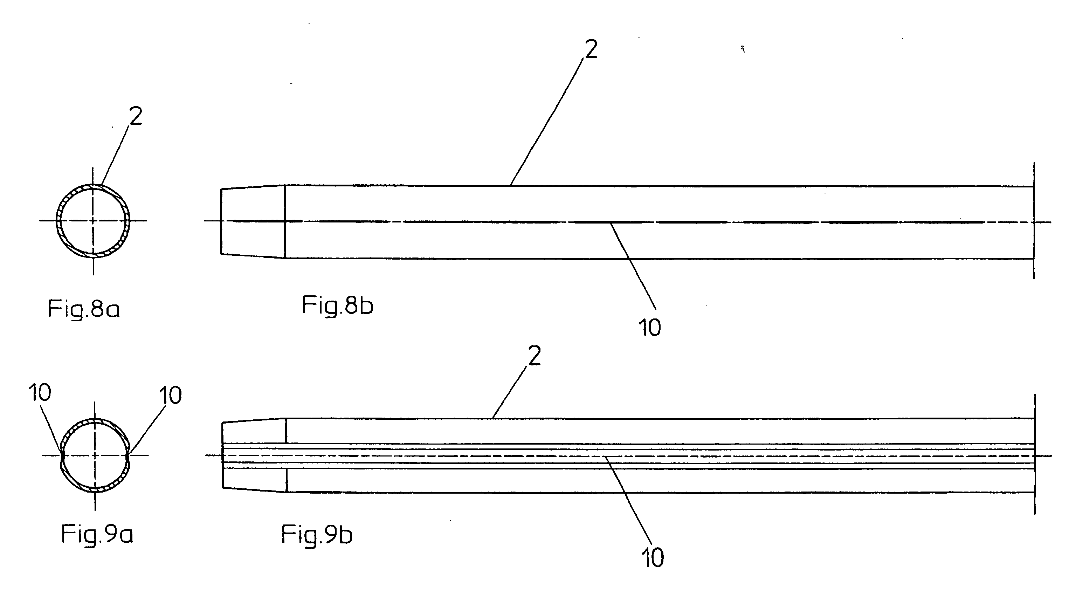

Fig. 8a und Fig. 8b zeigen ein erstes Ausführungsbeispiel der erfindungsgemäßen Führungshülse 2, wobei Fig. 8a eine Schnittansicht und Fig. 8b eine Seitenansicht zeigt. Die Führungshülse gemäß Fig. 8a und Fig. 8b weist über den Umfang eine konstante Materialstärke auf. An einer Seite ist eine sich in Längsrichtung der Führungshülse erstreckende Sollbruchstelle 10 in Form einer Perforation ausgebildet.

Fig. 9a und Fig. 9b zeigen eine zweite, alternative Ausführungsform der erfindungsgemäßen Führungshülse 2, wobei Fig. 9a eine Schnittansicht und Fig. 9b eine Seitenansicht zeigt. Bei dieser Ausführungsform ist die sich in Längsrichtung der Führungshülse 2 erstreckende Sollbruchstelle 10 in Form einer Schwächung der Wandstärke ausgebildet. Das bedeutet, die Führungshülse 2 weist im Bereich der Sollbruchstelle 10 eine geringe Wandstärke als in den übrigen Umfangsbereichen auf. Ferner weist diese Ausführungsform zwei diametral entgegengesetzt angeordnete Sollbruchstellen 10 am Umfang der Führungshülse 2 auf. Dies bewirkt, dass die Führungshülse 2 beim Aufweiten durch den Dilatationsdorn 20 in zwei Teile zerfällt. Vorzugsweise können entsprechend an den beiden Teilen jeweils ein Haltering 8 vorgesehen sein, um die beiden getrennten Teile der Führungshülse 2 leicht aus der Öffnung 16 proximalwärts herausziehen zu können. Ferner ist auch eine Kombination der Ausführungsformen gemäß Fig. 8a und Fig. 8b mit den Ausführungsformen gemäß Fig. 9a und Fig. 9b möglich. Das bedeutet, es kann eine Sollbruchstelle 10 geschaffen werden, welche zum einen eine dünnere Wandstärke und zum anderen gleichzeitig eine Perforation aufweist.

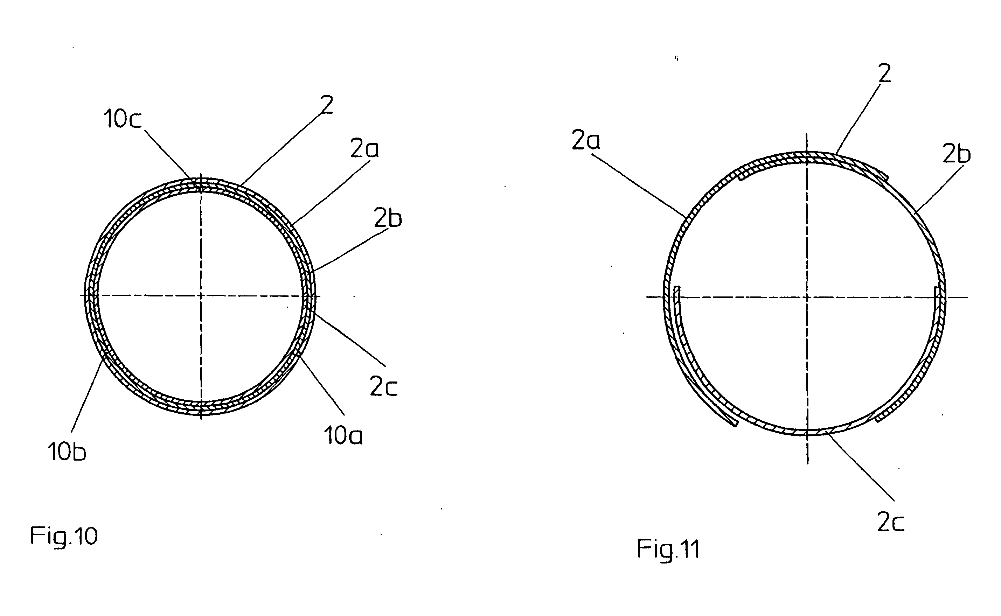

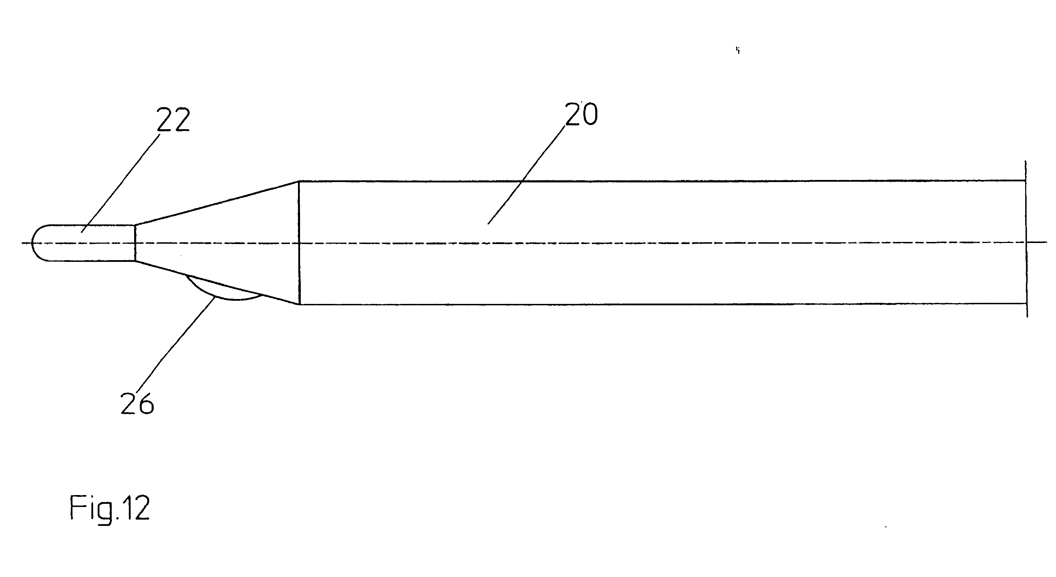

Anhand von Figuren 10 und 11 wird eine dritte Ausführungsform der erfindungsgemäßen Führungshülse 2 beschrieben. Figuren 10 und 11 zeigen Schnittansichten dieser Führungshülse 2, wobei die Führungshülse in Fig. 10 im geschlossenen Zustand und in Fig. 11 im aufgeweiteten Zustand gezeigt ist. Die Führungshülse gemäß Fig. 10 besteht aus drei ineinanderliegend angeordneten Hülsen 2a, 2b und 2c. Dabei weist die Hülse 2c einen Außendurchmesser auf, welcher im Wesentlichen dem Innendurchmesser der Hülse 2b entspricht, und die Hülse 2b weist einen Außendurchmesser auf, welcher im Wesentlichen dem Innendurchmesser der Hülse 2a entspricht. Dies ermöglicht, dass die Hülsen 2a, 2b und 2c aneinander liegend angeordnet sind. Dabei bilden die Hülsen 2a, 2b und 2c getrennte Teile, so dass sie in Umfangsrichtung gegeneinander beweglich bzw. verschiebbar sind. Jede der drei Hülsen 2a, 2b und 2c weist jeweils eine Sollbruchstelle oder einen Schlitz 10a, 10b bzw. 10c auf. Dabei sind die Sollbruchstellen 10a, 10b und 10c gleichmäßig verteilt um jeweils 120° versetzt zueinander über den Umfang der Führungshülse 2 verteilt angeordnet. Das bewirkt, dass jede Sollbruchstelle 10a, 10b und 10c jeweils von zumindest einer Wandung der jeweils anderen Hülsen überdeckt wird.

Fig. 11 zeigt die Führungshülse 2 gemäß Fig. 10 im aufgeweiteten Zustand, d. h. nach Einführung des Dilatationsdornes 20. Die Hülsen 2a, 2b und 2c weiten sich auf, wobei an den Stellen der Sollbruchstellen bzw. Schlitze 10a, 10b und 10c in jeder der Hülsen 2a, 2b und 2c umfängliche Spalte entstehen, welche jedoch jeweils von der Wandung einer der anderen Hülsen 2a, 2b und 2c überdeckt werden, so dass auch im aufgeweiteten Zustand eine umfänglich vollständig geschlossene Führungshülse 2 erhalten bleibt. Es gibt somit keine durchgehenden Öffnungen oder Spalte in der Führungshülse 2, durch welche Gewebe in das Innere der Führungshülse 2 eindringen kann.

Fig. 12 zeigt eine Seitenansicht einer bevorzugten Ausführungsform eines Dilatationsdornes 20. Der Dilatationsdorn 20 weist im Bereich seines kegelförmigen bzw. konischen Abschnittes eine Schneide 26 auf, welche sich in Längsrichtung des Dilatationsdornes 20 erstreckt und dazu dient, beim Einschieben des Dilatationsdornes 20 in die Führungshülse 2 diese aufzutrennen bzw. aufzuschneiden. Dabei kann die Führungshülse 2 an beliebiger Stelle des Umfangs aufgetrennt werden, auch wenn keine entsprechende Sollbruchstelle vorgesehen ist. Bevorzugt jedoch wird mit der Schneide 26 eine Sollbruchstelle 10 in der Führungshülse 2 aufgetrennt.

### Bezugszeichenliste

- 2 -: Führungshülse
- 2a, 2b, 2c -: Hülsen
- 4 -: Trokarhülse
- 6 -: Veresskanüle
- 8 -: Haltering
- 10 -: Sollbruchstelle
- 10a, 10b, 10c -: Sollbruchstellen oder Schlitze
- 12 -: Anschrägung
- 14 -: Gewebe
- 16 -: Öffnung
- 18 -: proximalseitige Öffnung
- 20 -: Dilatationsdorn
- 22 -: Führungsnase
- 24 -: Trokarhülse
- 26 -: Schneide

## Patentansprüche

1. Dilatationssystem bestehend aus einem Dilatationsdorn (20) mit einem Durchmesser, welcher vom distalen Ende proximalwärts zunimmt, und einer rohrförmigen Führungshülse (2), **dadurch gekennzeichnet, dass** die Führungshülse eigenstabil ausgebildet ist und einen Innendurchmesser aufweist, welcher dem Durchmesser des distalen im Durchmesser reduzierten Endes (22) des Dilatationsdornes (20) entspricht, und in ihrer Längsrichtung über die gesamte Länge entlang zumindest einer Linie (10) auftrennbar ist.

2. Dilatationssystem nach Anspruch 1, bei welchem die Führungshülse (2) zumindest eine sich in Längsrichtung der Führungshülse (2) vorzugsweise über deren gesamte Länge erstreckende Sollbruchstelle (10) aufweist.

3. Dilatationssystem nach Anspruch 1 oder 2 mit einer Veresskanüle (6), welche einen Außendurchmesser aufweist, der dem Innendurchmesser der Führungshülse (2) entspricht.

4. Dilatationssystem nach einem der vorangehenden Ansprüche, bei welchem in der Führungshülse (2) zwei vorzugsweise diametral entgegengesetzt angeordnete Sollbruchstellen (10) ausgebildet sind, welche sich in Längsrichtung der Führungshülse (2) erstrecken.

5. Dilatationssystem nach einem der Ansprüche 2 bis 4, bei welchem die Sollbruchstellen (10) durch Perforation oder bereichsweise schwächere Wandstärke der Führungshülse (2) ausgebildet sind.

6. Dilatationssystem nach einem der vorangehenden Ansprüche, bei welchem die Führungshülse (2) aus zumindest zwei voneinander getrennten, ineinander angeordneten Hülsen (2a, 2b, 2c) ausgebildet ist, welche jeweils zumindest einen sich in Längsrichtung der Führungshülse (2) vorzugsweise über deren gesamte Länge erstreckenden Schlitz oder eine sich entsprechend erstreckende Sollbruchstelle (10a, 10b, 10c) aufweisen, wobei die Schlitze oder Sollbruchstellen (10a, 10b, 10c) in den beiden Hülsen (2a, 2b, 2c) zueinander umfänglich versetzt angeordnet sind.

7. Dilatationssystem nach einem der vorangehenden Ansprüche, bei welchem am proximalen Ende der Führungshülse (2) zumindest ein Halteelement (8) ausgebildet ist.

8. Dilatationssystem nach einem der vorangehenden Ansprüche, bei welchem die Führungshülse (2) an ihrem distalen Ende verjüngt oder konisch ausgebildet ist.

9. Dilatationssystem nach einem der vorangehenden Ansprüche, bei welchem die Führungshülse (2) aus einem vorzugsweise transparenten Kunststoff gefertigt ist.

10. Dilatationssystem nach einem der vorangehenden Ansprüche, bei welchem der Dilatationsdorn (20) zumindest eine Schneide (26) zum Auftrennen der Führungshülse (2) aufweist.

## Claims

1. A dilatation system consisting of a dilatation pin (20) with a diameter which increases proximally from the distal end, and of a tubular guide sleeve (2), **characterised in that** the guide sleeve is inherently stable and has an inner diameter which corresponds to the diameter of the distal end of the dilatation pin (20), said end being reduced in diameter, and in its longitudinal direction may be split over the whole length along at least one line (10).

2. A dilatation system according to claim 1, with which the guide sleeve (2) comprises a predetermined break location (10) extending in the longitudinal direction of the guide sleeve (2) preferably over its whole length.

3. A dilatation system according to claim 1 or 2 with a veress cannula (6) which has an outer diameter which corresponds to the inner diameter of the guide sleeve (2).

4. A dilatation system according to one of the preceding claims, with which two predetermined break locations (10) arranged preferably in a diametrically opposed manner are formed in the guide sleeve (2), which extend in the longitudinal direction of the guide sleeve (2).

5. A dilatation system according to one of the claims 2 to 4, with which the predetermined break locations (10) are formed by perforation or a wall thickness of the guide sleeve (2) which is weaker in regions.

6. A dilatation system according to one of the preceding claims, with which the guide sleeve (2) consists of at least two sleeves (2a, 2b, 2c) which are arranged in one another and are separated from one another and which in each case comprise at least one slot extending in the longitudinal direction of the guide sleeve (2) preferably over the its whole length, or a correspondingly extending predetermined break location (10a, 10b, 10c), wherein the slots or predetermined break locations (10a, 10b, 10c) in the two sleeves (2a, 2b, 2c) are arranged peripherally offset to one another.

7. A dilatation system according to one of the preceding claims, with which at least one holding element (8) is formed at the proximal end of the guide sleeve (2).

8. A dilatation system according to one of the preceding claims, with which the guide sleeve (2) at its distal end is designed tapered or in a conical manner.

9. A dilatation system according to one of the preceding claims, with which the guide sleeve (2) is manufactured of a preferably transparent plastic.

10. A dilatation system according to one of the preceding claims, with which the dilatation pin (20) comprises at least one cutter (26) for splitting the guide sleeve (2).

## Revendications

1. Système de dilatation comprenant un mandrin de dilatation (20) dont le diamètre augmente depuis l'extrémité distale vers le côté proximal, et un manchon de guidage (2) de forme tubulaire, **caractérisé en ce que** le manchon de guidage est conçu en étant autostable et présente un diamètre intérieur qui correspond au diamètre de l'extrémité (22) distale, de diamètre réduit, du mandrin de dilatation (20), et peut être séparé dans sa direction longitudinale sur toute la longueur le long d'au moins une ligne (10).

2. Système de dilatation selon la revendication 1, dans lequel le manchon de guidage (2) présente au moins une zone de rupture obligatoire (10) s'étendant dans le sens longitudinal du manchon de guidage (2), de préférence sur toute sa longueur.

3. Système de dilatation selon la revendication 1 ou 2, avec une canule (6) de Veress, qui présente un diamètre extérieur correspondant au diamètre intérieur du manchon de guidage (2).

4. Système de dilatation selon l'une des revendications précédentes, dans lequel, dans le manchon de guidage (2), sont réalisés deux zones de rupture obligatoire (10) disposées de préférence de façon diamétralement opposée, qui s'étendent dans le sens longitudinal du manchon de guidage (2).

5. Système de dilatation selon l'une des revendications 2 à 4, dans lequel les zones de rupture obligatoire (10) sont réalisées par perforation ou par un affaiblissement par endroits de l'épaisseur de paroi du manchon de guidage (2).

6. Système de dilatation selon l'une des revendications précédentes, dans lequel le manchon de guidage (2) est réalisé à partir d'au moins deux manchons (2a, 2b, 2c) séparés les uns des autres et disposés les uns dans les autres, qui présentent chacun au moins une fente s'étendant dans le sens longitudinal du manchon de guidage (2) de préférence sur toute sa longueur ou une zone de rupture obligatoire (10a, 10b, 10c) s'étendant de manière correspondante, les fentes ou les zones de rupture obligatoire (10a, 10b, 10c) étant disposées dans les deux manchons (2a, 2b, 2c) en étant décalées sur le pourtour les unes par rapport aux autres.

7. Système de dilatation selon l'une des revendications précédentes, dans lequel au moins un élément de retenue (8) est prévu à l'extrémité proximale du manchon de guidage (2).

8. Système de dilatation selon l'une des revendications précédentes, dans lequel le manchon de guidage (2) est réalisé en étant rétréci ou conique à son extrémité distale.

9. Système de dilatation selon l'une des revendications précédentes, dans lequel le manchon de guidage (2) est fabriqué à partir d'une matière plastique de préférence transparente.

10. Système de dilatation selon l'une des revendications précédentes, dans lequel le mandrin de dilatation (20) présente au moins une lame (26) pour la séparation du manchon de guidage (2).
